Europäisches Patentamt

European Patent Office

Office européen des brevets

(11) Publication number: **0 264 333**
A2

# (12) EUROPEAN PATENT APPLICATION

(21) Application number: **87402300.5**

(22) Date of filing: **15.10.87**

(51) Int. Cl.⁴: **A 61 K 49/00**
A 61 K 33/00, A 61 K 47/00,
A 61 K 37/14, A 61 K 39/395

(30) Priority: **17.10.86 US 920808**

(43) Date of publication of application:
**20.04.88 Bulletin 88/16**

(84) Designated Contracting States:
**AT BE CH DE ES FR GB GR IT LI LU NL SE**

(71) Applicant: **CYTOGEN CORPORATION**
**201 College Road East Forrestal Research Center**
**Princeton New Jersey 08540 (US)**

(72) Inventor: **Siegel, Richard C.**
**6 Brown Court**
**Annandale New Jersey (US)**

**Alvarez, Vernon Leon**
**18 Rice Drive**
**Morrisville Pennsylvania (US)**

(74) Representative: **Ahner, Francis et al**
**CABINET REGIMBEAU 26, avenue Kléber**
**F-75116 Paris (FR)**

(54) Method for preparation of protein-chelator-metal ion compositions suitable for injection.

(57) This invention relates to an improved method for preparing protein-chelator-metal ion complex compositions suited for parenteral administration to human patients. Also described are the in vivo therapeutic and diagnostic applications for which the compositions prepared according to this method are advantageously used.

EP 0 264 333 A2

**Description**

METHOD FOR PREPARATION OF PROTEIN-CHELATOR-METAL ION COMPOSITIONS SUITABLE FOR INJECTION

1. FIELD OF THE INVENTION

The present invention relates generally to chelated metal ion compositions for in vivo administration to patients. More particularly, the invention relates to an improved method for preparing injectable compositions comprising protein-chelator-metal ion complexes which are substantially-free of non-chelated metal ions. The term "substantially free" of non-chelated metal ions is intended to mean that the compositions are at least about 90-95% free of metal ions associated with the protein, but not chelated or coordinately complexed with the chelator.

The protein-chelator-metal ion compositions prepared using the improved method of the invention are useful for in vivo diagnostic and therapeutic applications.

2. BACKGROUND OF THE INVENTION

Metal ions associated with the protein moiety of protein-chelator metal ion complexes but not coorindately complexed or chelated to the chelator have been termed "adventitiously bound" metal ions. Conventional methods for removing adventitiously bound metal ions have entailed lengthy dialysis procedures and/or column chromatographic separation using Sephadex, Chelex-100 and the like (see Khaw et al., Science 209: 295 (1980); U.S. Pat. No. 4,421,735 issued to Haber et al.).

U.S. Pat. No. 4,472,509 issued to Gansow et al. describes a method for removing such adventitiously bound metal from metal chelated conjugated monoclonal antibodies which requires use of one or more ion exchange, retardation or chelating resins in combination with elution through a sizing matrix such as polyacrylamide, agarose, or polysaccharide gel matrices. According to the method taught by Gansow et al., use of ion retardation or ion exchange resins alone is not sufficient. A sizing matrix (e.g. Sephadex G-50) in which the metal ions are retarded within the matrix on the basis of size while antibodies are not is also required to reduce the adventitiously bound metal ions to about 6% of the metal contained in the conjugate.

European Appln. No. 85401695.3 filed on August 29, 1985 describes the removal of non-chelated metal ions from antibody-chelator metal ion complexes by means of elution through a sizing resin (e.g. a Sephadex G-50 column) followed by elution through a high performance liquid chromatography (HPLC) column.

All of the above conventional methods suffer from a number of disadvantages particularly when the compositions must be prepared for administration in a clinical setting. The conventional methods require either lengthy separation procedures or elution through complex chromatographic columns.

3. SUMMARY OF THE INVENTION

The present invention is based on the discovery that filtration through a filter comprised of polyvinylidene difluoride having a pore size ranging from about 0.1-0.45 um and preferably about 0.22 um results in a protein-chelator-metal ion complex that is substantially free of metal ions which are not coordinately complexed or chelated to the chelator. The term "substantially free" of non-chelated metal ions is intended to mean that the compositions are at least about 90-95% free of metal ions associated with the protein, but not chelated or coordinately complexed with the chelator.

Based on this discovery, the present invention provides an improved method for preparing protein-chelator-metal ion complexes suitable for injection to human patients which has the following advantages over conventional methods of preparation: (a) does not require the use of column chromatographic procedures; and (b) does not require lengthy dialysis or elution procedures. Thus the present method is easier, faster and less complex than conventional methods. Such ease of preparation is particularly important in the clinical setting for which the compositions are intended. When using column chromatographic procedures in the clinic it is difficult to prepare sterile, non-pyrogenic materials suitable for parenteral administration to humans. The method of the present invention provides a particularly easy means for preparing such sterile, non-pyrogenic materials.

The method of the invention comprises passing a protein-chelator-metal ion complex in sterile buffered saline through a sterile filter comprised of polyvinylidene difluoride having a pore size ranging from about 0.1-0.45 um, preferably about 0.22 um and collecting the filtrate containing the protein-chelator-metal ion complex substantially free of non-chelated metal ions.

Alternatively, the method comprises mixing a protein-chelator conjugate in sterile buffered solution with a sterile solution of a metal ion in a chelating buffer, such as acetate or citrate, filtering the resulting mixture through a filter comprised of polyvinylidene difluoride having a pore size ranging from 0.1-0.45 um, preferably about 0.22 um and collecting the filtrate containing the protein-chelator metal-ion complex substantially free of non-chelated metal ions.

According to another alternate embodiment, the method of the invention further comprises incubating the protein-chelator-metal ion complex in sterile buffered saline with a sterile buffered saline solution of a chelator for about 10-15 minutes, before passing the protein-chelator-metal iron complex through the sterile filter.

Because the compositions prepared by the method of the invention are substantially free from non-chelated metal ions they are particularly useful for in vivo diagnostic and therapeutic applications in which the metal ions is intended to be delivered to a specific target site in the body.

The present invention may be more fully understood by reference to the following detailed description and examples of specific embodiments.

## 4. DETAILED DESCRIPTION OF THE INVENTION

The invention relates to an improved method for preparing protein-chalator-metal ion complexes suited for injection to human patients. As used throughout this application, the "protein" moiety functions to target the complex to a specific cell, tissue, organ or other site in the body. The term "protein" moiety is intended to encompass any proteinaceous substance that can be attached covalently to a chelator while sufficiently retaining its specific affinity for a target site to direct delivery of the complex to that site when administered in vivo. Glycoproteins such as antibodies and antibody fragments are typical examples of the "protein" moiety, but the method of the invention is not limited to antibody-chelator-metal ion complexes. Also encompassed are antigens, proteins such as receptor binding proteins and the like and proteins such as fibronectin which has specific affinity for collagenous sites, etc.

The chelator moiety encompasses any bifunctional chelating agent that is (a) able to donate electrons and combine by coordinate bonding with a metal ion to form a structure called a chelate or chelation complex and (b) suitable for attachment to a protein without loss of the ability to chelate metal ions and without destroying the ability of the protein to specifically react with the site for which it has affinity. Useful chelators include but are not limited to: diethylentriaminepentaacetic acid (DTPA), ethylendiaminetetraacetic acid (EDTA); dimercaptosuccinic acid; 2-3-dimercaptosuccinic acid, metallothioein, cryptates such as described by Gansow et al, J. Heterocyclic Chem. 18297 (1981), and derivatives of such chelators as described in the European Patent Application No. 85401695.3.

The protein and chelator moiety are covalently attached by methods known to those skilled in the art including but not limited to methods described by Rodwell et al., Proc. Nat'l Acad. Sci. USA 83: 2632 (1986); Krejcarek et al., Biophys. Res. Comm. 77: 581 (1977), etc. Particularly preferred methods are described in European Application No. 85401695.3.

According to the latter methods, an antibody-chelator conjugate may be first prepared and later combined with the metal ion of interest to form a protein-chelator-metal ion complex. Alternatively, a metal ion may be first coordinately bound to a chelator and the chelator-metal ion covalently attached to an antibody.

Independent of the method of attachment or preparation of a protein-chelator-metal ion complex, the improved method of the present invention results in the preparation of protein-chelator-metal ion complexes in which about 90-95% of the metal ion in the complex is chelated to the complex resulting in complexes advantageously useful for injection to patients.

The improved method of the invention comprises: passing a protein-chelator-metal ion complex in sterile buffered saline through a sterile filter comprised of polyvinylidene difluoride having a pore size ranging from about 0.1-0.45 um. preferably about 0.22 um and collecting the filtrate containing the protein-chelator-metal ion complex substantially free from non-chelated metal ions.

Alternatively, the method comprises: contacting a protein-chelator conjugate in sterile buffered solution with a sterile solution of a metal ion in a chelating buffer, such as acetate or citrate, to form a protein-chelator-metal ion complex; filtering the resulting mixture through a filter comprised of polyvinylidene difluoride having a pore size ranging from 0.1-0.45 um, preferably about 0.22 um and collecting the filtrate containing the protein-chelator metal ion complex substantially free of non-chelated metal ions.

According to another alternate embodiment, the method of the invention further comprises incubating the protein-chelator-metal ion complex in sterile buffered saline with a sterile buffered saline solution of a chelator for about 10-15 minutes, before passing the protein-chelator-metal ion complex through the sterile filter.

Although not desiring to be limited to any particular mechanism to explain the surprising results obtained using the novel method of the invention, Applicants point out that the filter comprised of polyvinylidene difluroide does not appear to function to exclude the non-chelated metal ions on the basis of size. Rather Applicants believe the metal ions interact with the filter by a process such as adsorption or the like.

## 4.1. USES FOR THE INJECTABLE COMPOSITIONS

The injectable compositions comprising a protein-chelator-metal ion complex substantially free of non-chelated metal prepared according to the method of the present invention are advantageously used for a number of in vivo diagnostic and therapeutic applications in which targeted delivery of the metal ion is desired. The metal ion is selected based upon its therapeutic effects for treating cellular disorders such as all neoplasms including carcinomas, adenomas and hyperplaysias; certain immunological disorders such as autoimmune diseases, immune suppressive diseases, etc. or for its ability to be detected using various imaging methods. The protein moiety of the composition functions to deliver the metal ion to the desired target site. For example, when the protein moiety is an antibody or antibody fragment the immunospecificity and immunoreactivity of such antibody molecule for a particular antigen directs the complex to specific cells, tissues, organs or any other site having the particular antigen. Alternatively, the protein moiety may be selected from any non-antibody protein molecules having selective affinity for specific cells, tissues, organs or other site.

Because the compositions prepared according to the invention are substantially free of non-chelated metal ions, the compositions are particularly useful for selective delivery of the metal ion to the intended target site without delivery to non-intended sites.

The concentration of the protein-chelator-metal ion complex in the compositions prepared according to the invention will depend upon the intended application and is well within the scope of knowledge of one of ordinary skill.

According to one intended therapeutic application, the metal ion is selected for its cell killing properties. Suitable beta-emitting metal ions for therapeutic uses include but are not limited to: Scandium-46, Scandium-47, Scandium-48, Gallium-72, and Gallium-73, Palladium-109, Yttrium-90 and Copper-67. Suitable alpha-emitting metals ions include those with a relatively short half-life of about 4 days or less including, but not limited to: Bismuth-211, Bismth-212, Bismuth-213 Bismuth-214, and Lead-212.

According to one intended diagnostic application, the metal ion is selected for its ability to be detected at an in vivo site by scintography. For this imaging application the metal ion is chosen from those radioactive metal ions having the following characteristics: (a) high photon yield to permit a single image to be obtained in several hours; (b) radiation exposure to the most critical organ of less than 5 rad; (c) isotope does not decay by emission of a particle; and (d) half-life of less than 4 days. Suitable radioactive metal ions include but are not limited to Indium-111, Indium 113m, Technetium-99m, etc.

An alternative method of imaging with radioisotopes involves positron emission tomography. Suitable positron emitting isotopes include Scandium-43, Scandium-44, Iron-52, Cobalt-55 and Gallium-48.

Tissue imaging may also utilize nonradioactive paramagnetic metal ions such as Iron-54, Iron-56, Iron-57, Iron-58, Gadolinium-157 and Maganese-55, which are detectable by nuclear magnetic resonance spectroscopy.

Tissues which one may image include any solid neoplasm, certain organs such as lymph nodes, parathyroids, spleen and kidney, sites of inflammation or infection (e.g., macrophages at such sites), myocardial infarction or thromboses (neoantigenic determinants on fibrin or platelets), etc.

The following Examples are given for purposes of illustration and not by way of limitation on the scope of the invention.

## 5. EXAMPLES: PREPARATION OF ANTIBODY-CHELATOR-IN-111 COMPOSITIONS SUITABLE FOR INJECTION

Throughout the following examples, the trivalent radioactive metal ion $^{111}$Indium$^{+3}$ is represented by "In-111."

## 5.1 METHOD OF PREPARATION OF ANTIBODY-GYK-DTPA-IN-111 COMPOSITION FOR INJECTION

Preliminary to preparing an antibody-GYK-DTPA-IN-111 complex for injection, the following compositions were prepared:

A tripeptide derivative of DEPA anhydride glycyl-tyrosyl-(N- ) lysine-DTPA (GYK-DTPA) was prepared according to methods described in European Application No. 85401695.3 as follows:

the initial reactant N-FMOC-glycyl-(o-benzyl-tyrosyl-( -B-carbobenzy)) lysine was prepared according to standard solid phase synthetic methods described by Barant and Merrifield in The Peptides, Vol. 2, Gross and Meienhoffer (ed.), Acad. Press, New York, pp. 3-385. The dertivatized peptide was cleaved from the resin and partially deblocked by bubbling hydrogen bromide (HBr) gas through a suspension of the reaction mixture in trifluoroacetic acid containing methoxybenzene (a fify-fold molar excess over tyrosine) for 60 minutes at room temperature. The resin was removed by filtration and the filtrate evaporated to dryness. The residue was dissolved in a minimum amount of methanol, and the product precipitated with ether and used without further purification.

One mole N-FMOC-glycyl-tryrosyl-lysine in dimethylformamide (DMF) was reacted with 1 mole DTPA mixed anhydride and maintained at room temperature for 1 hour. The solvent was removed by rotary evaporation, and the oily residue dissolved in a small aliquot of DMF. Distilled water was added to precipitate the FMOC-GYK-DTPA produced. The FMOC group was removed by addition of an equal volume of 40% dimethylamine to a solution of FMOC-GYK-DTPA in DMF for 30 minutes at room temperature. The solvent was evaporated to dryness and the residue taken up in distilled water. The crude product was purified by extraction with ethyl acetate, and the resulting aqueous solution of GYK-DTPA was lyophilized to dryness.

A murine monoclonal antibody S4 IgG$_{2a}$ was covalently attached to GYK-DTPA according to methods described in '328 application. Briefly, the oligosaccharide moiety of the S4 antibody was oxidized by incubation in the dark with 10 mM NaIO$_4$ in phosphate buffered saline (PBS, 0.15 M NaCl, 0.01 M PO $\frac{=}{4}$) pH 6.0 for one hour on ice. The oxidized antibody was then eluted through a sephadex G-25 column equilibriated with PBS to remove excess reagents. Oxidized S4 antibody was then incubated with a two-thousand fold molar excess of GYK-DTPA and 10 mM sodium cyanoborohydride (Aldrich Chemical Co., Milwaukee, Wisconsin) for 16-24 hours at room temperature. The reaction mixture was eluted through a gel filtration chromatographic column to purify the monomeric antibody-GYK-DTPA conjugate.

A sterile solution of the S4 antibody-GYK-DTPA conjugate, in PBS adjusted to pH 6.0 with sodium hydroxide or hydrochloric acid was prepared by filtration.

A sterile solution of DTPA, 10 mM in PBS_(3.9 mg/ml DTPA) pH adjusted to 5.8 using sodium hydroxide or hydrochloric acid was obtained by filtration.

One ml of sterile aqueous sodium acetate solution, 0.5 M, (68.0 mg sodium acetate trihydrate ml sterile water, adjusted to pH 6.0 using glacial acetic acid) was added to an equal volume of a commercially available sterile solution of In-111 chloride (Amersham, Arlington Heights, Illinois) to form buffered In-111 solution.

A sterile injectable antibody-GYK-DTPA-In-111 composition was prepared according to the method of the invention as follows:

Buffered In-111 solution prepared as above was mixed with 0.4-40 ml (depending upon the concentration of antibody desired) of sterile S4-GYK-DTPA solution and the mixture incubated for about 60 minutes at 37°C to form the antibody-chelator-metal ion complex S4-GYK-DTPA-In-111. Then 2.0 ml of sterile DTPA solution was added and the mixture incubated for 10 minutes at room temperature. The mixture was then passed through a sterile polyvinylidene difluoride filter having a pore size of 0.22 um (Millex-GVO25LS, Millipore, Bedford, Massachusetts) and collecting the filtrate comprising the sterile antibody-chelator-metal ion substantially free of non-chelated metal ions. The entire filtration process required no more than 1-5 minutes.

## 5.2. EVALUATION OF NON-CHELATED IN-111 IN ANTIBODY-GYK-DTPA-IN-111 COMPOSITION

The amount of non-chelated or adventitiously associated In-111 in the injectable antibody-GYK-DTPA-In-111 prepared according to the present invention was evaluated using HPLC as follows:

Preparations of antibody-GYK-DTPA-In-111 complexes were analyzed by liquid chromatography employing a TSK-G3000 SW column (Toyo Soda, Japan) and an on-line radioactivity detector. Preparations analyzed in this manner showed greater than 95% of the total In-111 associated with the protein-chelator complex.

Control experiments were conducted to quantitate the radioactivity associated with the filters comprised of polyvinylidene difluoride treated with either free or chelated In-111. Greater than 90% of the chelated In-111 passed through the filter while only 6% of the free In-111 was eluted from the filter. These control experiments indicate that the efficiency of the filter to remove free In-111 is greater than 94%.

The invention described and claimed herein is not to be limited in scope by the specific embodiments herein disclosed, since these embodiments are intended as illustrations of several aspects of the invention. Any equivalent embodiments are intended to be within the scope of this invention. Indeed, various modifications of the invention in addition to those shown and described herein will become apparent to those skilled in the art from the foregoing description. Such modifications are also intended to fall within the scope of the appended claims.

## Claims

1. A method for preparing a protein-chelator-metal ion complex composition suitable for injection, comprising:

(a) passing a protein-chelator-metal ion complex in sterile buffered saline through a sterile filter comprised of polyvinylidene difluoride having a pore size ranging from about 0.1-0.45 um; and

(b) collecting the filtrate containing the protein-chelator metal ion complex substantially free of non-chelated metal ions.

2. The method according to claim 1 in which the protein used to form the protein-chelator-metal in ion complex is an antibody or an antibody fragment.

3. The method according to claim 1 in which the chelator is selected from the group consisting of diethylenetriaminepetaacetic acid; ethylenediaminetetracetic acid; dimercaptosuccinic acid; 2-3-dimercaptosuccinic acid; metallothioein, cryptates and derivatives thereof.

4. The method according to claim 1 in which the filter has a pore size of abut 0.22 um.

5. The method according to claim 1, further comprising briefly incubating a protein-chelator-metal ion complex in sterile buffered saline with a sterile buffered solution of a chelator prior to step (a).

6. A method for preparing a protein-chelator-metal ion complex composition suitable for injection, comprising:

(a) mixing a protein-chelator conjugate in sterile buffered saline with an aliquot of a sterile solution of a metal ion in a chelating buffer for about 60 minutes at about 24-37°C to form a protein-chelator-metal ion complex

(b) filtering the mixture through a filter comprised of polyvinylidene difluoride having a pore size ranging from about 0.1-0.45 um; and

(c) collecting the filtrate containing the protein-chelator-metal ion complex substantially free of non-chelated metal ions.

7. The method according to claim 6 in which the protein used to form the protein-chelator-metal in ion complex is an antibody or an antibody fragment.

8. The method according to claim 6 in which the chelator is selected from the group consisting of diethylenetriaminepetaacetic acid; ethylenediaminetetracetic acid; dimercaptosuccinic acid; 2-3-dimercaptosuccinic acid; metallothioein, cryptates and derivatives thereof.

9. The method according to claim 6 in which the filter has a pore size of abut 0.22 um.

10. The method according to claim 6, in which the chelating buffer is selected from the group consisting of acetate and citrate buffer, adjusted to pH about 5.5-6.5.

11. The method according to claim 6, further comprising briefly incubating the protein-chelator-metal ion complex in sterile buffered saline with a sterile buffered solution of a chelator prior to step (b)